# EUROPEAN PATENT SPECIFICATION

(11) **EP 0 641 862 B1**
(45) Date of publication and mention of the grant of the patent: **12.12.2001**
(21) Application number: 94113955.2
(22) Date of filing: 06.09.1994
(51) Int. Cl.: C12P 13/24, C12N 9/02

(54) **Process for producing trans-4-hydroxy-L-proline**
Verfahren zur Herstellung von Trans-4-Hydroxy-L-Prolin
Procédé de préparation de trans-4-hydroxy-L-proline

(30) Priority: 07.09.1993 JP 22194093; 27.12.1993 JP 33256193
(43) Date of publication of application: 08.03.1995
(73) Proprietor: KYOWA HAKKO KOGYO CO., Ltd., Chiyoda-ku, Tokyo 100 (JP)
(72) Inventor: Ozaki, Akio, Machida-shi, Tokyo (JP); Mori, Hideo, Machida-shi, Tokyo (JP); Shibasaki, Takeshi, Machida-shi, Tokyo (JP); Ando, Katsuhiko, Machida-shi, Tokyo (JP); Chiba, Shigeru, Kawasaki-shi, Kanagawa (JP)
(74) Representative: Vossius, Volker, Dr.

(56) References cited:
- EP-A- 0 190 350
- EP-A- 0 547 898
- EP-A- 0 555 475
- CHEMICAL ABSTRACTS, vol. 66, no. 13, 27 March 1967 Columbus, Ohio, US; abstract no. 52480, HUTTON, JOHN J., JR. ET AL: "Cofactor and substrate requirements of collagen proline hydroxylase" XP002029266 & ARCH. BIOCHEM. BIOPHYS. (1967), 118(1), 231-40 CODEN: ABBIA4, 1967,
- Arch.Biochem.Biophys.118,611-618(1967)
- Arch.Biochem.Biophys 144, 343-351(1971)

### SEQUENCE LISTING

(1) GENERAL INFORMATION:
   (i) APPLICANT:
      (A) NAME: KYOWA HAKKO KOGYO CO., LTD.
      (B) STREET: 6-1, Ohtemachi 1-chome, Chiyoda-ku
      (C) CITY: Tokyo
      (E) COUNTRY: Japan
      (F) POSTAL CODE (ZIP): none
      (G) TELEPHONE: 0081-3-3201-7211
      (H) TELEFAX: 0081-3-3284-1968
      (I) TELEX: J24543HKKYOWA
   (ii) TITLE OF INVENTION: PROCESS FOR PRODUCING TRANS-4-HYDROXY-L-PROLINE
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 27 amino acids
      (B) TYPE: amino acid
      (D) TOPOLOGY: unknown
   (ii) MOLECULE TYPE: protein
   (v) FRAGMENT TYPE: N-terminal
   (xi) SEQUENCE DESCRIPTION: SEQ ID NO: 1:
   Met Leu Thr Pro Thr Glu Leu Lys Gln Tyr Arg Glu Ala Gly Tyr Leu
   1               5                   10                  15
   Leu Ile Glu Asp Gly Leu Gly Pro Arg Arg Val
               20                  25

## Description

### Background of the Invention

The present invention relates to a process for producing trans-4-hydroxy-L-proline. Trans-4-hydroxy-L-proline is useful as a starting compound for medicines and an additive to foods. The present invention also relates to a novel enzyme capable of catalyzing the hydroxylation of L-proline at the 4-position of L-proline. The novel enzyme is used in the above-mentioned process.

There have heretofore been known microbiological processes of producing trans-4-hydroxy-L-proline as follows:
1) A method of producing trans-4-hydroxy-L-proline from 4-hydroxy-2-oxoglutaric acid using microorganisms belonging to the genus Escherichia (Japanese Published Unexamined Patent Application No. 266995/91),
2) A method of producing trans-4-hydroxy-L-proline using fungi (EP 0547898 A2), and
3) A method of producing trans-4-hydroxy-L-proline by enzymatically hydroxylating L-proline using microorganisms belonging to the genus Streptomyces (J. Biol. Chem., 254, 6684 - 6690, 1979; Biochem. Biophys. Res. Comm., 120, 45 - 51, 1984).

Chemical Abstracts, Vol. 66 no. 524 80 p & Arch. Biochem. Biophys., Vol. 118(1) 1967, pp 231-240 discloses proline hydroxylase from animal tissues which catalyses the reaction of peptidyl proline to peptidylhydroxyproline.

The methods are not suitable in the industrially application because in the first method, 4-hydroxy-2-oxoglutaric acid is expensive and the productivity of trans-4-hydroxy-L-proline is extremely low; in the second method the productivity of trans-4-hydroxy-L-proline is extremely low, and in the third method, the enzymatic activity is so weak that the final product could not be detected without using a radioactive compound.

Heretofore, an enzyme capable of catalyzing the hydroxylation of L-proline into trans-4-hydroxy-L-proline (hereinafter referred to as L-proline-4-hydroxylase) has not been isolated. A process for producing trans-4-hydroxy-L-proline, using an enzyme source which is isolated from a microorganism belonging to the genus Dactylosporangium or Amycolatopsis and which catalyzes the hydroxylation of L-proline into trans-4-hydroxy-L-proline, has not been known. Although there was a paper reporting that L-proline-4-hydroxylase was isolated from a microorganism belonging to the genus Streptomyces (Tetrahedron Letters, 34, 7489 - 7492, 1993), the report is silent about steps for isolating the enzyme, the enzyme purity, the physicochemical properties of the enzyme, etc.

The object of the present invention is to provide an efficient process for the production of trans-4-hydroxy-L-proline on the industrially applicable basis, and the second object of the present invention is to provide a novel enzyme which catalyzes the hydroxylation of L-proline at the 4-position of L-proline and is useful in the above process.

### Summary of the Invention

The present invention provides a process for the production of trans-4-hydroxy-L-proline which comprises allowing L-proline to coexist with 2-ketoglutaric acid, a divalent iron ion and an enzyme source which catalyzes hydroxylation of L-proline at the 4-position of L-proline in an aqueous medium to convert L-proline into trans-4-hydroxy-L-proline, and recovering the trans-4-hydroxy-L-proline from the aqueous medium. The present invention further provides a novel hydroxylase having the following physicochemical properties(hereinafter referred to as L-proline-4-hydroxylase):

### (1) Activity and Substrate Specificity:

The enzyme catalyzes the hydroxylation of L-proline at the 4-position of L-proline in the presence of 2-ketoglutaric acid and a divalent iron ion to produce trans-4-hydroxy-L-proline.

### (2) Optimum pH Range:

The enzyme has an optimum pH range of 6.0 to 7.0 for its reaction at 30°C for 20 minutes.

### (3) Stability pH Range:

The enzyme is stable at pH values of 6.5 to 10.0, when it is allowed to stand at 4°C for 24 hours.

### (4) Optimum Temperature Range:

The optimum temperature range is from 30 to 40°C when it is allowed to stand at pH 6.5 for 15 minutes.

### (5) Stable Temperature Range:

The enzyme is inactivated, when it is allowed to stand at pH 9.0 and at 50°C for 30 minutes.

### (6) Inhibitors:

The activity of the enzyme is inhibited by metal ions of Zn⁺⁺ and Cu⁺⁺ and ethylenediaminetetraacetic acid.

### (7) Activation:

The enzyme does not need any cofactors for its activity.

L-Ascorbic acid accelerates the activity of the enzyme.

### (8) Km Value:

Km value is 0.27 mM for L-proline and is 0.55 mM for 2-ketoglutaric acid, when determined in a 80 mM 2-(N-morpholino)ethanesulfonic acid (MES) buffer (pH 6.5) containing 4 mM L-ascorbic acid, 2 mM ferrous sulfate and the enzyme preparation.

### (9) Molecular Weight:

The enzyme has a molecular weight of 32,000 ± 5,000 daltons by sodium dodecylsulfate-polyacrylamide gel electrophoresis and of 43,800 ± 5,000 daltons by gel filtration.

### (10) N-terminal Amino Acid Sequence:

The enzyme has an N-terminal amino acid sequence illustrated by Sequence Number 1 mentioned below.

### Detailed Description of the Invention

As the enzyme source to be used in the process for producing trans-4-hydroxy-L-proline of the present invention, any microorganism can be used as far as it has an enzymatic activity of catalyzing the hydroxylation of L-proline at the 4-position in the presence of 2-ketoglutaric acid and a divalent iron ion. Examples of microorganisms having such activity are microorganisms belonging to the genus Dactylosporangium or Amycolatopsis. The preferred strains of such microorganisms include Dactylosporangium sp. RH1 and Amycolatopsis sp. RH2. Specifically, a culturer cells or processed cells of these : strains can be used. Further, a crude enzyme preparation from cells of the microorganisms as mentioned above, a purified product of such enzyme preparation, an immobilized enzyme preparation, etc. can be used.

The strains RH1 and RH2 were newly isolated by the present inventors from a tree in Tokyo, Japan and from the soil in Saitama, Japan, respectively. The bacteriological properties of the strains RH1 and RH2 are described below.

### 1. Morphology:

The morphological properties when the strains were cultivated on various media at 28°C for 14 days are shown in Table 1.

**Table 1 -**

| Morphological Properties | | |
|---|---|---|
| | Strain RH1 | Strain RH2 |
| 1) Hyphae | | |
| Branching mode of hyphae | Simple branching | Simple branching |
| Formation of aerial hyphae | Not observed | Yes |
| Fragmentation of aerial hyphae | | Yes |
| Fragmentation of substrate hyphae | Not observed | Yes |

| 2) Spores | | |
|---|---|---|
| Sporulation | Observed (as sporangiospores) | Not observed |
| Positions to which spores adhere | Adhered to substrate hyphae | |
| Morphology of sporangia | Rods, existing singly or as bundles composed of several rod sporangia. | Not observed |
| Number of sporangiospores per one sporangium | 2 to 4 | |
| Characteristics of sporangiospores | | |
| Surface | Smooth | |
| Shape | Oval sphere | |
| Size | 0.6 to 0.8 µm × 1.0 to 2.0 µm | |
| Motility | Yes | |

| 3) Others | | |
|---|---|---|
| Globose bodies | Observed | Not observed |
| Pseudosporangia | Not observed | Not observed |
| Chlamydospores | Not observed | Not observed |
| Synnema | Not observed | Not observed |

### 2. Culture Characteristics in Various Media:

The strain RH1 grows normally or vigorously on usual synthetic and natural media, while its substrate hyphae are orange. On some media, the strain often produces ocher or pale red soluble pigments.

The strain RH2 grows normally or vigorously on usual synthetic and natural media, while its substrate hyphae are pale yellow or brown and its aerial hyphae are white or gray. On some media, the strain often produces brown soluble pigments.

The characteristics in the growth conditions and the colors of the strain RH1 and the strain RH2, when the strains were cultivated on various media at 28°C for 14 days, are shown in Table 2. The designation of the colors has been made, according to the classification of colors indicated in Color Harmony Manual published by Container Corporation of America.

**Table 2 -**

| Culture Characteristics in Various Media | | |
|---|---|---|
| | Strain RH1 | Strain RH2 |
| 1) Sucrose-nitrate agar | | |
| Growth | Moderate | Poor |
| Color of substrate hyphae | Apricot (4ga) | Pearly pink (3ca) |
| Formation of aerial hyphae | None | Poor |
| Color of aerial hyphae | | White (a) |
| Soluble pigments | None | None |
| 2) Glucose-asparagine agar | | |
| Growth | Moderate | Moderate |
| Color of substrate hyphae | Light gray to luggage tan (c to 4ne) | Light tan (3gc) |
| Formation of aerial hyphae | None | Poor |
| Color of aerial hyphae | | White to light amber (a to 3ic) |
| Soluble pigments | None | Produced only a little (ocher) |
| 3) Glycerol-asparagine agar | | |
| Growth | Poor | Moderate |
| Color of substrate hyphae | Light melon yellow (3ea) | Light white to cinnamon (2ea to 31e) |
| Formation of aerial hyphae | None | Moderate |
| Color of aerial hyphae | | White (a) |
| Soluble pigments | None | Produced only a little (ocher) |
| 4) Inorganic salt-starch agar | | |
| Growth | Moderate | Moderate |
| Color of substrate hyphae | Camel (3ie) | Light mustard tan to cinnamon (2ic to 31e) |
| Formation of aerial hyphae | None | Moderate |
| Color of aerial hyphae | | White to light gray (a to d) |
| Soluble pigments | None | Produced only a little (ocher). |
| 5) Tyrosine agar | | |
| Growth | Moderate | Moderate |
| Color of substrate hyphae | Apricot (4ia) | Mustard gold to golden brown |
| | | (2pg to 3pg) |
| Formation of aerial hyphae | None | None |
| Color of aerial hyphae | | White (a) |
| Soluble pigments | Produced only a little (pale red) | Produced (brown) |
| 6) Nutrient agar | | |
| Growth | Poor | Poor |
| Color of substrate hyphae | Bright melon yellow (3ia) | Yellow maple (3ng) |
| Formation of aerial hyphae | None | Moderate |
| Color of aerial hyphae | | Light gray (b) |
| Soluble pigment | Produced only a little (ocher) | Produced (brown) |
| 7) Yeast extract-malt extract agar | | |
| Growth | Abundant | Good |
| Color of substrate hyphae | Orange (41a) | Mustard gold to golden brown (2pg) to (3pg) |
| Formation of aerial hyphae | Not formed | Moderate |
| Color of aerial hyphae | | Pearl (2ba) |
| Soluble pigments | Produced only a little (ocher) | Produced (brown) |
| 8) Oatmeal agar | | |
| Growth | Poor or moderate | Poor |
| Color of substrate hyphae | Apricot (4ia) | Mustard gold (2pg) |
| Formation of aerial hyphae | None | Poor |
| Color of aerial hyphae | | Natural (3dc) |
| Soluble pigments | Produced only a little (ocher) | Produced only a little (ocher) |

### 3. Physiological Properties:

The physiological properties of the strains RH1 and RH2 are shown in Table 3, in which the "temperature range for growth" indicates the results of each strain after 7 day-cultivation by shaking. The remaining items indicate the results after 2 to 3 week-cultivation at 28°C.

**Table 3 -**

| Physiological Properties | | |
|---|---|---|
| | Strain RH1 | Strain RH2 |
| 1) Temperature Range for Growth | 20 to 37°C | 13 to 35°C |
| 2) Liquefaction of Gelatin | - | + |
| 3) Hydrolysis of Starch | + | + |
| 4) Coagulation of Skim Milk Powder | - | + |
| 5) Peptonization of Skim Milk Powder | - | + |
| 6) Formation of Melanoid Pigment | | |
| (1) Peptone-yeast extract-iron agar | - | + |
| (2) Tyrosine agar | + | + |
| 7) Utilization of Carbon Sources(*) | | |
| L-Arabinose | + | - |
| D-Glucose | + | + |
| D-Xylose | + | + |
| Sucrose | + | (+) |
| Raffinose | (+) | + |
| D-Fructose | + | + |
| Rhamnose | + | + |
| Inositol | (+) | + |
| D-Mannitol | + | + |

| | | |
|---|---|---|
| (*) Pridham Gottlieb-agar medium was used as basic medium. + indicates that the strain utilized the carbon source; - indicates that the strain did not utilize the carbon source; and (+) indicates that it is not clear whether or not the strain utilized the carbon source. | | |

### 4. Chemotaxonomic Properties:

The chemotaxonomic properties of the strains RH1 and RH2 are shown in Table 4.

**Table 4 -**

| Chemotaxonomic Properties | | |
|---|---|---|
| | Strain RH1 | Strain RH2 |
| 1) Configuration of diaminopimelic acid in cell wall | 3-OH (meso) form | Meso form |
| 2) Reducing sugars in hydrolysate of cell wall | | Galactose and minor amount of arabinose |
| 3) Reducing sugars in hydrolysate of whole cell | Arabinose and xylose | Arabinose and galactose |
| 4) Mycolic acid | | Not contained |
| 5) Phospholipid | | The cells contain phosphatidylethanolamine but do not contain phosphatidylcholine and unknown glucosamine-containing phospholipids. |
| 6) Menaquinone | | The cells contain MK-9 (H₄) as the major component. |

Accordingly, the strain RH1 is identified as the genus Dactylosporangium of actinomycetes in view of its properties (1) that it does not form aerial hyphae, (2) that it forms rod-like sporangia on its substrate hyphae, (3) that its sporangia contain from 2 to 4 motile spores each, (4) that the diaminopimelic acid contained in its cell wall is 3-hydroxydiaminopimelic acid and (5) that the hydrolysate of its whole cell contains reducing sugars of arabinose and xylose.

The strain RH2 is identified as the genus Amycolatopsis of actinomycetes in view of its properties (1) that it forms aerial hyphae, (2) that its aerial hyphae and substrate hyphae fragmented into coccoid or rod-shaped elements, (3) that its cell wall contains meso-diaminopimelic acid and reducing sugars of galactose and a slight amount of arabinose, (4) that the hydrolysate of its whole cell contains reducing sugars of arabinose and galactose, (5) that it does not contain mycolic acid as its cell-constitutive component, (6) that it contains a phospholipid of phosphatidylethanolamine but does not contain phosphatidylcholine and unknown glucosamine-containing phospholipids and (7) that the major component of its major menaquinone is MK-9 (H₄).

The strain RH1 was named Dactylosporangium sp. RH1 and the strain RH2 was named Amycolatopsis sp. RH2. These strains have been deposited with National Institute of Bioscience and Human-Technology, Agency of Industrial Science and Technology in Japan as of September 1, 1993 under FERN BP-4400 for the strain RH1 and as of February 22, 1994 under FERM BP-4581 for the strain RH2, both in terms of the Budapest Treaty.

The medium for cultivating these microorganisms may be any of natural media and synthetic media, so long as it contains carbon sources, nitrogen sources, inorganic salts, etc. that may be assimilated by microorganisms having an activity of catalyzing hydroxylation of L-proline to produce trans-4-hydroxy-L-proline.

As the carbon sources, carbohydrates such as glucose, fructose, sucrose, molasses containing these components, starch and starch hydrolysates, etc.; organic acids such as acetic acid, propionic acid, etc.; alcohols such as ethanol, propanol, etc. may be used.

As the nitrogen sources, ammonia; ammonium salts of various inorganic acids and organic acids such as ammonium chloride, ammonium sulfate, ammonium acetate, and ammonium phosphate, etc.; other nitrogen-containing compounds; peptone, meat extracts, yeast extracts, corn steep liquor, casein hydrolysates, soy bean cakes, soy bean cake hydrolysates, various cultured cells of microorganisms, their digested products, etc. may be used.

The inorganic material includes, for example, potassium dihydrogen phosphate, dipotassium hydrogen phosphate, magnesium phosphate, magnesium sulfate, sodium chloride, ferrous sulfate, manganese sulfate, copper sulfate, calcium carbonate, etc.

The cultivation of these microorganisms is carried out under aerobic conditions, for example, by shaking culture or submerged-aerial stirring culture. The temperature for the cultivation is preferably from 15 to 37°C, and the period for the cultivation is generally from 16 to 96 hours. During the cultivation, the pH of the medium is kept at 5.0 to 9.0 with inorganic or organic acids, alkaline solutions, urea, calcium carbonate, ammonia, etc.

The amount of the enzyme source to be used in the process for producing trans-4-hydroxy-L-proline depends on the amount of the substrate to be used in the process. Usually, it may be from 1.0 to 10,000,000 U, preferably from 1,000 to 2,000,000 U per one liter of the aqueous medium.

The enzyme activity for producing one nmol of trans-4-hydroxy-L-proline for one minute under the conditions mentioned below is defined as one unit (U).

The enzyme preparation to be determined is added to 80 mM MES buffer (pH 6.5) containing 4 mM L-proline, 8 mM 2-ketoglutaric acid, 2 mM ferrous sulfate and 4 mM L-ascorbic acid to make 250 µl in total, and the mixture was allowed to stand at 30°C for 20 minutes. The reaction mixture is heated at 100°C for 2 minutes so as to stop the reaction, and the amount of the trans-4-hydroxy-L-proline produced in the reaction mixture is determined by HPLC.

For the determination, any method capable of determining the amount of trans-4-hydroxy-L-proline may be employed. For instance, generally usable are a post-column derivatization method where HPLC is utilized, and a pre-column derivatization method where the compound to be determined in the reaction mixture is previously reacted with 7-chloro-4-nitrobenz-2-oxa-1,3-diazole chloride (hereinafer referred to as NBD) to form its NBD-derivative, the derivative is separated by reversed-phase chromatography using HPLC and the thus-separated derivative is quantitatively determined by spectrofluorometry (excitation wavelength: 503 nm, emission wavelength: 541 nm). The pre-column derivatization method may be conducted, according to the method of William J. Lindblad & Robert F. Diegelmann, et al. (see Analytical Biochemistry, Vol. 138, pp. 390 - 395, 1984).

The concentration of L-proline to be used for producing trans-4-hydroxy-L-proline may be from 1 mM to 2 M.

The process for producing trans-4-hydroxy-L-proline needs a divalent iron ion. The concentration of the divalent iron ion may be generally from 1 to 100 mM. Any divalent, iron; ion may be used so long as it does not interfere with the enzymatic reaction. For instance, sulfides such as ferrous sulfate, chlorides such as ferrous chloride, ferrous carbonate, the salts of organic acids such as citrates, lactates, fumarates, etc. may be used.

The process also needs 2-ketoglutaric acid. The concentration of 2-ketoglutaric acid is generally from 1 mM to 2M. 2-Ketoglutaric acid itself may be added to the aqueous medium, or alternatively, a compound that may be converted into 2-ketoglutaric acid by the metabolic activity of the microorganism used in the enzymatic reaction may be added thereto. The compound includes, for example, saccharides such as glucose, glutamic acid, succinic acid, etc. These compounds may be used singly or in combination.

The aqueous medium to be used in the process of the present invention includes, for example, water, phosphates, carbonates, acetates, borates, citrates, buffers such as tris-buffers, alcohols such as methanol and ethanol, esters such as ethyl acetate, ketones such as acetone, amides such as acetamide, etc.

The enzymatic reaction may be carried out in the culture medium where the above-mentioned microorganisms having an activity of catalyzing hydroxylation of L-proline to produce trans-4-hydroxy-L-proline are being cultivated or have been cultivated, or alternatively, the enzymatic reaction may also be carried out in an aqueous medium containing the cells of the above mentioned microorganisms separated from the culture, processed cells, or a purified or crude enzyme derived from the cells.

Processed cells of the microorganisms include, for example, dried cells, lyophilized cells, surfactant-treated cells, enzymatically-treated cells, ultrasonically-treated cells, mechanically-ground cells, mechanically-compressed cells, solvent-treated cells, cellular protein fractions, immobilized cells, etc.

The enzymatic reaction is generally carried out at a temperature of 15 to 50°C and at a pH of 6.0 to 9.0, for a period of 1 to 96 hours. If desired, surfactants and/or organic solvents may be added during the processing of the cells or during the enzymatic reaction.

As the surfactants, mention may be made of cationic surfactants such as polyoxyethylene-stearylamine (e.g., Nymeen S-215, made by Nippon Oils and Fats Co.), cetyltrimethylammonium bromide, Cation FB, Cation F2-40E, etc.; anionic surfactants such as sodium oleylamidosulfate, Newrex TAB, Rapizole 80, etc.; ampholytic surfactants such as polyoxyethylene-sorbitan monostearate (e.g., Nonion ST221), etc; other tertiary amines PB, hexadecyldimethylamine, etc. Any surfactant that may promote the reaction may be employed. The concentration of the surfactant to be employed in the reaction may be generally from 0.1 to 50 mg/ml, preferably from 1 to 20 mg/ml.

As the organic solvent, mention may be made of toluene, xylene, aliphatic alcohols, benzene, ethyl acetate, etc. Generally, the concentration of the solvent in the process may be from 0.1 to 50 µl/ml, preferably from 1 to 20 µl/ml.

To recover trans-4-hydroxy-L-proline from the aqueous medium, ordinary separation methods such as column chromatography using ion-exchange resins, crystallization, etc. may be employed. The recovered trans-4-hydroxy-L-proline may be identified by ordinary analytic means using, for example, ¹³C-NMR spectrum, ¹H-NMR spectrum, mass spectrum, specific rotatory power, etc.

Next, the novel enzyme, the L-proline-4-hydroxylase of the present invention is described below.

The hydroxylase may be obtained by cultivating microorganisms having an ability to produce L-proline-4-hydroxylase in a medium so as to produce and accumulate the hydroxylase in the culture medium, and collecting the hydroxylase from the cells.

Any microorganisms having an ability to produce L-proline-4-hydroxylase may be employed. For example, microorganisms belonging to the genus Dactylosporangium or Amycolatopsis and having such activity can be used. Specific examples are the above-mentioned Dactylosporangium sp. RH1 and Amycolatopsis sp. RH2, a subcultivated strain thereof, a mutant thereof, a derivative thereof, etc.

The medium for cultivating these microorganisms may be any of natural media and synthetic media, so long as it contains carbon sources, nitrogen sources, inorganic salts, etc. that may be assimilated by microorganisms having an ability to produce L-proline-4-hydroxylase.

The carbon source includes, for example, carbohydrates such as glucose, fructose, sucrose, molasses containing these compounds, starch and starch hydrolysates, etc.; organic acids such as acetic acid, propionic acid, etc.; alcohols such as ethanol, propanol, etc. which may be assimilated by the microorganisms.

As the nitrogen source, ammonia; ammonium salts of various inorganic acids and organic acids such as ammonium chloride, ammonium sulfate, ammonium acetate, ammonium phosphate, etc.; other nitrogen-containing compounds; peptone, meat extracts, yeast extracts, corn steep liquor, casein hydrolysates, soy bean cakes, soy bean cake hydrolysates, various microorganisms for fermentation, their digested products, etc. may be used.

As the inorganic material, dipotassium hydrogen phosphate, potassium dihydrogen phosphate, magnesium phosphate, magnesium sulfate, sodium chloride, ferrous sulfate, manganese sulfate, copper sulfate, calcium carbonate, etc. can be used.

The cultivation of these microorganisms is effected under aerobic conditions, for example, by shaking culture or submerged-aeration stirring culture. The temperature of the cultivation is preferably from 15 to 37°C, and the period for the cultivation is generally from 16 to 96 hours.

During the cultivation, the pH of the medium is kept at 5.0 to 9.0. The pH of the medium is adjusted with inorganic or organic acids, alkaline solutions, urea, calcium carbonate, ammonia, etc. During the cultivation, L-proline may be added, if desired.

To isolate and purify the enzyme from the culture containing the enzyme, any ordinary method for isolating and purifying an enzyme may be employed. For instance, the culture is subjected to centrifugation to collect the cultivated cells therefrom, and the cells are fully washed and then disrupted by an ultrasonic cell disruptor, a French press, a Manton-Gauline homogenizer, a Dyno mill, etc. to obtain a cell-free extract. The cell-free extract is again subjected to centrifugation, and the resulting supernatant is then purified, for example, by salting-out with ammonium sulfate or the like, by anion-exchange chromatography with diethylaminoethyl (DEAE)-Sepharose or the like, by hydrophobic chromatography with butyl-Sepharose, phenyl-Sepharose or the like, by dye affinity chromatography with red-Agarose or the like, by gel filtration with molecular sieves or by electrophoresis such as isoelectric point electrophoresis or the like. In this way, a pure product of the enzyme is obtained. The activity of the hydroxylase thus isolated may be determined by the same method as mentioned above.

The L-proline-4-hydroxylase, obtained according to the manner mentioned above, has the following physicochemical properties (1) to (10):

### (1) Activity and Substrate Specificity:

The hydroxylase catalyzes hydroxylation of L-proline at the 4-position of L-proline in the presence of 2-ketoglutaric acid and a divalent iron ion to form trans-4-hydroxy-L-proline.

### (2) Optimum pH Range:

In the above-mentioned method of determining the activity of the hydroxylase, the reaction was carried out while the buffer component in the reaction mixture was changed to sodium acetate buffer at pH of 3.5 to 5.5, to MES buffer at pH of 5.5 to 6.5, to TES buffer [N-tris(hydroxymethyl)methyl-2-aminoethanesulfonic acid] at pH of 7.0 to 7.5, to TAPS buffer [N-tris(hydroxymethyl)methyl-3-aminopropanesulfonic acid] at pH of 8.0 to 9.0, and to CAPSO buffer (3-N-cyclohexylamino-2-hydroxypropanesulfonic acid) at pH of 9.5 to 11.0. As a result, it was found that the optimum pH range for the enzyme was pH 6.0 to pH 7.0.

### (3) Stability pH Range:

The enzyme was allowed to stand in the presence of 50 mM of a buffer (sodium acetate buffer at pH of 3.5 to 5.5, MES buffer at pH of 5.5 to 6.5, TES buffer at pH of 7.0 to 7.5, TAPS buffer at pH of 8.0 to 9.0, CAPSO buffer at pH of 9.5 to 11.0), 2 mM dithiothreitol (DTT) and 20% (v/v) of glycerol, at 4°C for 24 hours, and then its activity was determined. The enzyme kept at pH ranging from 6.5 to 10.0 had an activity of 90% or more of the original activity of the enzyme. Accordingly, it was found that the enzyme was stable at pH ranging from 6.5 to 10.0.

### (4) Optimum Temperature Range:

In the above-mentioned method of determining the activity of the hydroxylase acting on the 4-position of L-proline, the activity of the enzyme was determined, varying the temperature. As a result, it was found that the enzyme had an optimum temperature ranging from 30 to 40°C in the reaction at pH of 6.5 for 15 minutes.

### (5) Stability Temperature Range:

When the enzyme was allowed to stand at 50°C and at pH of 9.0 for 30 minutes, it was inactivated.

### (6) Inhibitors:

The activity of the enzyme is inhibited by metal ions of Zn⁺⁺ and Cu⁺⁺ and by EDTA. According to the above-mentioned method of determining the activity of the hydroxylase acting on the 4-position of L-proline, when the activity of the enzyme was determined in the presence of Cu⁺⁺ and Zn⁺⁺ ions of 1 mM each, it was lowered to 13% and 6%, respectively, of the original activity of the enzyme. Likewise, when the activity of the enzyme was determined in the presence of 5 mM EDTA, no activity of the enzyme was detected.

### (7) Activation:

No activation of the enzyme was observed, when various metal ions and cofactors were added thereto. Accordingly, the enzyme does not need any cofactor for its activity.

Ascorbic acid promotes the activity of the enzyme.

### (8) Km Value:

The Km values of the enzyme were 0.27 mM for L-proline and was 0.55 mM for 2-ketoglutaric acid, when determined in the reaction system containing 80 mM MES buffer (pH 6.5), 4 mM L-ascorbic acid, 2 mM ferrous sulfate and a pre-determined amount of the enzyme.

### (9) Molecular Weight:

The molecular weight of the enzyme was calculated to be 32,000 ± 5,000 daltons, by sodium dodecylsulfate-polyacrylamide gel electrophoresis (using polyacrylamide gel, PAGEL NPU-12.5L (made by Atto Co.) and Molecular Weight Standard Broad Range SDS-PAGE (made by Biorad Co.). It was calculated to be 43,800 ± 5,000 daltons, by gel filtration with HPLC (using a column of G-3000SW having a size of 21.5 mm × 60 cm, and a molecular weight marker for HPLC made by Oriental Yeast Co. as the standard).

### (10) N-terminal Amino Acid Sequence:

The enzyme has an N-terminal amino acid sequence illustrate by Sequence Number 1.

The present invention is described by the following examples. However, the Examples are not intended to restrict the scope of the present invention.

### Example 1

### Production of Trans-4-hydroxy-L-proline:

SR3 medium comprising 1.0% glucose, 1.0% soluble starch, 0.5% yeast extract, 0.5% tryptone, 0.3% meat extract and 0.05% magnesium phosphate was adjusted at pH 7.2 with 6N-NaOH, put in test tubes (diameter 25 mm × length 200 mm) in an amount of 10 ml each and sterilized at 120°C for 20 minutes. One loopful of cells of Dactylosporangium sp. RH1, that had grown in HT-agar plate medium comprising 1% soluble starch, 0.2% NZ amine, 0.1% yeast extract, 0.1% meat extract and 1.5% agar, adjusted at pH 7.2 with 6N-NaOH, and sterilized at 120°C for 20 minutes, was inoculated into the above-mentioned medium in each test tube, cultivated at 28°C for 2 days by shaking, and used as a seed culture in the following steps.

Separately, Df1 medium comprising 5% soluble starch, 1.5% soybean meal, 0.05% monopotassium phosphate, 0.05% magnesium sulfate 7-hydrate and 0.5% calcium carbonate, and adjusted to pH 7.0 with 6N-NaOH, was put in test tubes (diameter 25 mm × length 200 mm) in an amount of 10 ml each and sterilized at 120°C for 20 minutes. One ml of the above-mentioned seed culture was inoculated in the medium in each test tube under germ-free condition and cultivated at 28°C for 2 days by shaking culture. The thus-obtained culture was centrifuged at 7000 × g for 10 minutes at 4°C. The cells thus separated were washed with 80 mM TES buffer (pH 7.5) and then. recentrifuged. 150 mg of the thus-obtained wet cells was suspended in 1.5 ml of a reaction mixture which had been prepared by adding 1.4% (v/v) of Nymeen solution [prepared by adding 4g of Nymeen S-215 (made by Nippon Oils & Fats Co.) to 10 ml of xylene] to 80 mM TES buffer (pH 7.5) containing 4 mM L-proline, 8 mM 2-ketoglutaric acid, 4 mM L-ascorbic acid and 2 mM ferrous sulfate and the mixture was allowed to stand at 30°C for 2 hours to carry out the enzymatic reaction.

After the reaction, the cells were removed from the reaction mixture by centrifugation, and hydroxyprolines produced in the supernatant were determined.

The determination was conducted by HPLC under the conditions mentioned below. To identify the intended product, the resulting product was eluted from the column and reacted with NBD in the column line to form its NBD-derivative and the derivative was determined by fluorophotometry. Conditions for determination by HPLC

### [1] Apparatus Used:

High Performance Liquid Chromatography Device (made by Shimadzu Seisakusho K.K.)

| | |
|---|---|
| Chromatopac | CR6A |
| System controller | SCL-6B |
| Autoinjector | SIL-6B |
| Liquid Chromatograph Pump | LC-6A |
| Column Oven | CTO-6A |
| Chemical Reaction Box | CRB-6A |
| Spectrofluorometric Detector | RF-550A |

### [2] Column Used:

SUMCHIRAL OA5000 (diameter 4.5 mm × length 250 mm, made by Sumika Chemical Analysis Service Limited)

### [3] Conditions for Analysis:

| | | |
|---|---|---|
| 1) | Mobile Phase: aqueous solution of copper sulfate | 1 mM |
| 2) | Flow Rate of Mobile Phase: | 1.0 ml/min |
| 3) | Column Temperature: | 38°C |
| 4) | Buffer: | 0.3M boric acid buffer, pH 9.6 with 25 mM ethylenediaminetetraacetic acid |
| 5) | Flow Rate of Buffer: | 0.2 ml/min |
| 6) | NBD Chloride Solution: | methanol solution of 0.5 g/liter |
| 7) | Flow Rate of NBD Chloride Solution: | 0.5 ml/min |
| 8) | Reaction Temperature: | 60°C |
| 9) | Reaction Time: | about 3 min |
| 10) | Wavelength for Detection: | |
| | excitation wavelength | 503 nm |
| | emission wavelength | 541 nm |
| 11) | Sample: | 10 µl |

As a result of the determination, it was verified that 249 µM (32.6 mg/liter) of trans-4-hydroxy-L-proline was produced in the reaction mixture.

### Example 2

### Purification of Trans-4-hydroxy-L-proline:

The seed cultivation was carried out in the same manner as in Example 1, using 2 liter-Erlenmeyer flasks each containing 200 ml of SR3 medium. Dfl medium was put in 5 liter-jar fermenters in an amount of 2 liters each and sterilized at 120°C for 20 minutes. The seed culture was inoculated in the Dfl medium in each jar fermenter under germ-free conditions and cultivated under 700 rpm and 1 vvm, at 28°C for 2 days. During the cultivation, the pH of the medium was not adjusted. The thus-obtained culture medium was centrifuged at 7,000 × g for 10 minutes at 4°C to obtain wet cells in an amount of 75g per liter of the culture. A part of the wet cells was washed with a physiological saline at 4°C, recentrifuged and freezed at -80°C. The freezed cells were stored at -80°C until just before use. Separately, 400g of the wet cells was suspended in 2 liters of the reaction mixture described in Example 1, put in a 3 liter-beaker, and the enzymatic reaction was carried out at 30°C for 4 hours with stirring.

After the reaction, the cells were removed from the reaction mixture by centrifugation, and hydroxyprolines produced in the supernatant were determined. It was verified that 480 µM (63.0 mg/liter) of trans-4-hydroxy-L-proline was produced in the reaction mixture.

The reaction mixture was adjusted at pH 4.5, and the supernatant separated from the reaction mixture was passed through a column filled with 200 ml of an ion-exchange resin, Diaion SKlB (NH₄⁺ type, made by Mitsubishi Kasei Corp.). The fraction containing trans-4-hydroxy-L-proline was concentrated under reduced pressure and then passed through a column filled with 20 ml of an ion-exchange resin, Diaion PA412 (OH-type, made by Mitsubishi Kasei Corp.). The fraction containing trans-4-hydroxy-L-proline was concentrated under reduced pressure, and adjusted at pH 9.6. 10 volume % of o-phthal aldehyde (hereinafter referred to as OPA) solution (0.075 g/ml ethanol solution) and 2 volume % of β-mercaptoethanol solution (10% v/v aqueous solution) were added thereto. The mixture was then allowed to stand at 60°C for 5 minutes, whereby impurities of primary amino acids contained therein were reacted with OPA. The resulting mixture was passed through a column filled with 10 ml of Sepabeads SP207 (made by Mitsubishi Kasei Corp.), to separate trans-4-hydroxy-L-proline from the impurities of OPA-derivatized primary amino acids. The fraction containing trans-4-hydroxy-L-proline was concentrated under reduced pressure and again passed through a column filled with 20 ml of an ion-exchange resin, Diaion PA412 (OH-type, made by Mitsubishi Kasei Corp.) to separate a fraction containing trans-4-hydroxy-L-proline. The final fraction was concentrated and dried to obtain 78 mg of white crystals of trans-4-hydroxy-L-proline. The yield of the product was 62%. The white crystals were analyzed, and the data of its ¹³C-NMR spectrum, ¹H-NMR spectrum, mass spectrum and specific rotation were found to coincide with a standard sample of trans-4-hydroxy-L-proline (made by Nacalai Tesque Co.).

### Example 3

### Production of Trans-4-hydroxy-L-proline:

SR3 medium was put in test tubes (diameter 25 mm × length 200 mm) in an amount of 10 ml each and sterilized at 120°C for 20 minutes. One loopful of cells of Amycolatopsis sp. RH2 that had grown in HT-agar plate medium was inoculated in the above-mentioned medium in each test tube, cultivated at 28°C for 2 days by shaking, and used as a seed culture in the following steps.

Separately, Dfl medium was put in test tubes (diameter 25 mm × length 200 mm) in an amount of 10 ml each and sterilized at 120°C for 20 minutes. One ml of the seed culture was inoculated in the medium in each test tube under germ-free conditions and cultivated at 28°C for 2 days by shaking. The thus-obtained culture was centrifuged at 7000 × g for 10 minutes at 4°C. The cells thus separated were washed with 100 mM TES buffer (pH 7.5) and then recentrifuged. 100 mg of the thus-obtained wet cells was suspended in 1.5 ml of a reaction mixture which had been prepared by adding 1.4% (v/v) of Nymeen solution [prepared by adding 4g of Nymeen S-215 (made by Nippon Oils & Fats Co.) to 10 ml of xylene] to 100 mM TES buffer (pH 7.5) containing 5 mM L-proline, 5 mM 2-ketoglutaric acid, 5 mM L-ascorbic acid and 1 mM ferrous sulfate and the mixture was allowed to stand at 30°C for 3 hours.

After the reaction, the cells were removed from the reaction mixture by centrifugation, and hydroxyprolines produced in the supernatant were determined.

The determination of trans-4-hydroxy-L-proline was conducted in the same manner as in Example 1.

It was verified that 25 µM (3.3 mg/liter) of trans-4-hydroxy-L-proline was produced in the reaction mixture.

### Example 4

### Purification of Trans-4-hydroxy-L-proline:

The seed cultivation was carried out in the same manner as in Example 3, using 2 liter-Erlenmeyer flasks each containing 200 ml of SR3 medium. Dfl medium was put in 5 liter-jar fermenters in an amount of 2 liters each and sterilized at 120°C for 20 minutes. The seed culture was inoculated in the Df1 medium in each jar fermenter under germ-free conditions and cultivated under 700 rpm and 1 vvm, at 28°C for 2 days.. During the cultivation, the pH of the medium was not adjusted. The thus-obtained culture medium was centrifuged at 7,000 × g for 10 minutes at 4°C to obtain wet cells in an amount of 75g per liter of the culture. Four hundred grams of the wet cells were suspended in 2 liters of the reaction mixture described in Example 3, and put in a 3 liter-beaker, and the reaction mixture was allowed to stand at 30°C for 4 hours with stirring.

The supernatant was adjusted at pH 4.5, and passed through a column filled with 200 ml of an ion-exchange resin, Diaion SKlB (NH₄⁺ type, made by Mitsubishi Kasei Corp.). The fraction containing trans-4-hydroxy-L-proline was concentrated under reduced pressure and then passed through a column filled with 20 ml of an ion-exchange resin, Diaion PA412 (OH-type, made by Mitsubishi Kasei Corp.). The fraction containing trans-4-hydroxy-L-proline was concentrated under reduced pressure, adjusted at pH 9.6, and 10 volume % of OPA solution (0.075 g/ml ethanol solution) and 2 volume % of β-mercaptoethanol solution (10% v/v aqueous solution) were added thereto. The mixture was then allowed to stand at 60°C for 5 minutes, whereby impurities of primary amino acids contained therein were reacted with OPA. The resulting mixture was passed through a column filled with 10 ml of Sepabeads SP207 (made by Mitsubishi Kasei Corp.), to separate trans-4-hydroxy-L-proline from the impurities of the OPA-derivatized primary amino acids. The fraction containing trans-4-hydroxy-L-proline was concentrated under reduced pressure and again passed through a column filled with 20 ml of an ion-exchange resin, Diaion PA412 (OH-type, made by Mitsubishi Kasei Corp.) to separate a fraction containing trans-4-hydroxy-L-proline. The final fraction was concentrated and dried to obtain 4.8 mg of white crystals of trans-4-hydroxy-L-proline. The yield of the product was 62%.

The white crystals were analyzed, and the data of their ¹³C-NMR spectrum, ¹H-NMR spectrum, mass spectrum and specific rotation were found to coincide with those of a standard sample of trans-4-hydroxy-L-proline (made by Nacalai Tesque Co.).

### Example 5

### Isolation and Purification of L-proline-4-hydroxylase:

(1) Preparation of Cell-free Extract: Six hundred grams of the freezed cells obtained in Example 2 were thawed up and suspended in 3 liters of Buffer A [50 mM TAPS buffer (pH 9.0) containing 2 mM DTT, 0.2 mM EDTA and 20% (v/v) of glycerol] while cooling with ice. The resulting suspension was milled by a Dyno-Mill (made by Willy A Bachofen Maschinenfabrik, Basel, Switzerland) to disrupt the cells. The debris was centrifuged at 6,500 × g at 4°C for 30 minutes to separate the supernatant.

The subsequent procedures were carried out under cooling with ice at a Temperature of 4°C or lower.

### (2) Isolation and purification by Column Chromatography:

### (2)-1 STREAMLINE:

The supernatant obtained in the previous step was passed through a STREAMLINE™ (made by Pharamacia Co.) filled with 300 ml of DEAE adsorbent that had been equilibrated with Buffer A, whereupon a fraction containing the L-proline-4-hydroxylase was eluted with Buffer A containing 0.3M NaCl.

### (2)-2 DEAE Sepharose Column Chromatography:

The active fraction obtained in the previous step was diluted three times with Buffer A and passed through a DEAE Sepharose column (5 cm × 15 cm) that had been equilibrated with Buffer A. The column was washed with Buffer A, and the fraction containing the enzyme was eluted with Buffer A having a linear concentration gradient of NaCl of 0 to 0.3M.

### (2)-3 Butyl Sepharose Column Chromatography:

NaCl was added to the active fraction obtained in the previous step until the NaCl concentration was 3M. The mixture was passed through a butyl Sepharose column (Butyl Sepharose 4 Fast Flow, 2.6 cm × 13 cm) that had been equilibrated with Buffer A containing 3M NaCl. The enzyme was stepwise eluted with four kinds of buffers each having a different NaCl concentration, Buffer A containing 3M NaCl, Buffer A containing 1.98M NaCl, Buffer A containing 0.99M NaCl and Buffer A containing no NaCl, in this order or from the eluent buffer having a higher NaCl concentration to that having a lower NaCl concentration.

### (2)-4 Phenyl Sepharose Column Chromatography:

NaCl was added to the active fraction obtained in the previous step until the concentration of NaCl was 3M. The mixture was passed through a phenyl Sepharose column (Phenyl Sepharose HP Hiload 16/10, 1.6 cm × 10 cm) that had been equilibrated with Buffer A containing 3M NaCl. The column was washed with Buffer A containing 3M NaCl, and the fraction containing the enzyme was eluted with Buffer A.

### (2)-5 Dye Affinity Column Chromatography:

The active fraction obtained in the previous step was de-salted, using a FD-10 column (made by Pharmacia Co.), and the resulting fraction was passed through a Reactive Red 120 column (1 cm × 12.7 cm; made by Sigma Co.) that had been equilibrated with Buffer A. The column was washed with Buffer A, and the fraction containing the enzyme was eluted with Buffer A having a linear concentration gradient of NaCl of 0 to 1.5M.

### (2)-6 Resource Q Column Chromatography:

The active fraction obtained in the previous step was de-salted, using a PD-10 column (made by Pharmacia Co.) that had been equilibrated with Buffer B [50 mM TAPS buffer (pH 8.0) containing 2 mM DTT, 0.1% (v/v) of Tween 20 and 20% (v/v) of glycerol], and the resulting fraction was passed through a RESOURCE™ Q column (1 ml; made by Pharmacia Co.) that had been equilibrated with Buffer B. The fraction containing the enzyme was eluted with Buffer B having a linear concentration gradient of NaCl of 0 to 0.2M.

The summary of the isolation and purification of the L-proline-4-hydroxylase is shown in Table 5.

**Table 5**

| Summary of Isolation and Purification of L-proline-4-hydroxylase | | | | |
|---|---|---|---|---|
| Fraction | Total Protein (mg) | Total Activity (U) | Relative Activity (U/Mg of protein) | Yield (%) |
| Cell-free Extract | 13,330 | 11,000 | 0.83 | 100 |
| Streamline | 4,875 | 4,880 | 1.00 | 44.4 |
| DEAE Sepharose | 353 | 3,820 | 10.8 | 34.7 |
| Butyl Sepharose | 35.1 | 1,310 | 37.3 | 11.9 |
| Phenyl Sepharose | 1.44 | 814 | 565.3 | 7.4 |
| Color Affinity | 0.212 | 366 | 1,726 | 3.3 |
| Resource Q | 0.100 | 384 | 3,840 | 3.5 |

### Example 6

### Properties of L-proline-4-Hydroxylase:

### (1) Analysis by Electrophoresis:

The purified enzyme preparation obtained in Example 5 was analyzed by sodium dodecylsulfate-polyacrylamide gel electrophoresis (using polyacrylamide gel PAGEL NPU-12.5L made by Atto Co. and SDS-PAGE Molecular Weight Standard, Broad Range made by Biorad Co.). It was verified that the enzyme was composed of almost homogeneous sub-units having a molecular weight of about 32,000 ± 5,000 daltons.

### (2) Properties Relating to Enzyme Reaction:

The enzyme was subjected to substrate omission and addition tests using reaction mixtures mentioned below, to investigate the compounds indispensable for the enzyme reaction of hydroxylating the 4-position of L-proline, the promoters for the reaction and the inhibitors against the reaction.

The reaction mixture was composed of 80 mM TES buffer (pH 7.5), 4 mM L-proline, 8 mM 2-ketoglutaric acid, 2 mM ferrous sulfate, 4 mM L-ascorbic acid, 2 mg/ml catalase and a pre-determined amount of the pure enzyme, the total volume being 500 µl. The reaction was initiated by addition of the enzyme and continued for 15 minutes at 30°C. The reaction was stopped by heating the reaction mixture at 100°C for 2 minutes. The amount of trans-4-hydroxy-L-proline formed in the reaction mixture was determined by the pre-column derivatization method. One hundred microliters of 0.3M boric acid buffer (pH 10.7), 4 µl of aqueous solution of 10% (v/v) mercaptoethanol and 16 µl of ethanol solution of 5% (w/v) OPA were added to 100 µl of the reaction mixture, and the mixture was allowed to stand at 60°C for 30 seconds. In addition, 50 µl of ethanol solution of 2% (w/v) NBD chloride was added thereto and the mixture was allowed to stand at 60°C for 40 minutes. The reaction was stopped by adding 30 µl of 1 N-hydrochloric acid to the reaction mixture, and the precipitates formed were removed by centrifugation and filtration. The trans-4-hydroxy-L-proline formed by the reaction was quantitatively determined by HPLC analysis.

The HPLC for the determination was carried out under the following conditions:

| | |
|---|---|
| Mobile Phase | 10 mM Citric Acid (pH 4.0)/Methanol = 3/1 v/v) |
| Flow Rate | 1 ml/min |
| Column | YMC Pack ODS AQ-312 (made by YMC Co., 6 × 150 mm) |
| Column Temperature | 50°C |
| Detection | Fluorophotometry (excitation wavelength: 503 nm, emission wavelength: 541 nm) |

L-proline, 2-ketoglutaric acid and Fe⁺⁺ ion are indispensable for the enzyme reaction of hydroxylating the 4-position of L-proline. L-Ascorbic acid promotes the reaction Zn⁺⁺ ion, Cu⁺⁺ ion and EDTA inhibit the reaction.

The test results are shown in Table 6.

**Table 6**

| Investigation of Components Influencing Enzyme Raction of Hydroxylase | | |
|---|---|---|
| Components in Reaction Mixture | Added (+)²⁾ Not Added (-) | Relative Activity³⁾ |
| Basic Reaction Mixture¹⁾ | | 100 |
| | - Pure Enzyme | 0 |
| | - L-proline | 0 |
| | - 2-Ketoglutaric Acid | 0 |
| | - Fe⁺⁺ | 0 |
| | - L-Ascorbic Acid | 51 |
| | - Catalase | 93 |
| | + 5 mM EDTA | 0 |
| | + 1 mM Zn⁺⁺ | 6 |
| | + 1 mM Cu⁺⁺ | 13 |

| | | |
|---|---|---|
| 1) The standard reaction mixture was 80 mM TES buffer (pH 7.5) containing 4 mM L-proline, 8 mM 2-ketoglutaric acid, 2 mM ferrous sulfate, 4 mM L-ascorbic acid, 2 mg/ml catalase and a pre-determined amount of the pure enzyme, the total volume being 500 µl. The reaction was carried out at 30°C for 15 minutes. | | |
| 2) "(+)" means that the reaction mixture contained the compound shown in the table. "(-)" means that the reaction mixture did not contain the compound shown in the table. The concentration shown in the table means the concentration of the component in the reaction mixture. | | |
| 3) The activity is indicated as relative activity to the activity in the standard reaction mixture of being 100. | | |

### (3) Optimum pH Range:

In the above-mentioned method of determining the enzyme activity of the L-proline-4-hydroxylase, the reaction was conducted while the buffer component in the reaction mixture was changed to sodium acetate buffer at pH of 3.5 to 5.5, to MES buffer at pH of 5.5 to 6.5, to TES buffer at pH of 7.0 to 7.5, to TAPS buffer at pH of 8.0 to 9.0, and to CAPSO buffer at pH of 9.5 to 11.0. As a result, the enzyme had an activity of more than 90% of the maximum activity thereof at pH falling within the range of pH 6.0 to pH 7.0. The detailed test results are shown in Table 7.

**Table 7**

| Optimum pH Range for the Enzyme Reaction | |
|---|---|
| pH | Relative Activity |
| 3.5 | 2.9 |
| 4.0 | 4.4 |
| 4.5 | 5.9 |
| 5.0 | 10.3 |
| 5.5 | 41.2 |
| 6.0 | 97.0 |
| 6.5 | 100 |
| 7.0 | 91.2 |
| 7.5 | 75.0 |
| 8.0 | 47.1 |
| 8.5 | 44.0 |
| 9.0 | 29.4 |
| 9.5 | 7.4 |
| 10.0 | 4.4 |
| 10.5 | 0 |
| 11.0 | 0 |

### (4) Stability pH Range:

The enzyme was kept in the presence of 50 mM of a buffer (sodium acetate buffer at pH of 3.5 to 5.5, MES buffer at pH of 5.5 to 6.5, TES buffer at pH of 7.0 to 7.5, TAPS buffer at pH of 8.0 to 9.0, CAPSO buffer at pH of 9.5 to 11.0), 2 mM DTT and 20% (v/v) of glycerol, at 4°C for 24 hours, and then its activity was determined. The enzyme kept at pH ranging from 6.5 to 10.0 had an activity of 90% or more of the original activity of the enzyme before the test. Accordingly, the enzyme was kept stable at pH ranging from 6.5 to 10.0.

### (5) Optimum Temperature Range:

In the above-mentioned method of determining the enzyme activity of the L-proline-4-hydroxylase, the reaction was carried out at temperatures ranging from 15 to 50°C for 15 minutes. As a result, the enzyme had an activity of 90% or more of the maximum activity thereof at temperatures ranging from 30 to 40°C. The detailed test results are shown in Table 8.

**Table 8**

| Optimum Temperature Range Reaction for the Enzyme Reaction | |
|---|---|
| Temperature (°C) | Relative Activity¹⁾ |
| 15 | 28 |
| 20 | 41 |
| 25 | 60 |
| 30 | 91 |
| 35 | 100 |
| 40 | 96 |
| 45 | 68 |
| 50 | 32 |

| | |
|---|---|
| 1) The Activity is indicated as a relative activity to the maximum activity of being 100. | |

### (6) Stability Temperature Range:

The enzyme was kept in 50 mM TAPS buffer (pH 9.0) containing 2 mM DTT, 0.1% (v/v) of Tween 20 and 20% (v/v) of glycerol at a temperature ranging from 0 to 60°C for 30 minutes and thereafter the activity of the enzyme was determined. As a result, the enzyme was inactivated at 50°C or higher for 30 minutes.

### (7) N-terminal Amino Acid Sequence:

Using the Protein Sequencer Model PPSQ-10 (made by Shimadzu Seisakusho K.K.) the N-terminal amino acid sequence of the enzyme was determined. The result was as follows:

### Example 7

### Production of Trans-4-hydroxy-L-proline:

The enzyme reaction with purified L-proline-4-hydroxylase was carried out. The reaction mixture was composed of 200 mM MES buffer (pH 6.5), 20 mM L-proline, 20 mM 2-ketoglutarate, 5 mM L-ascorbic acid, 2 mM ferrous sulfate, and 90U of purified enzyme preparation in a total volume of 50 µl. The reaction was carried out at 35°C for 30 min. As a result of the reaction, 12.9 mM (1.7 g/l) of trans-4-hydroxy-L-proline was produced in the reaction mixture.

## Claims

1. A process for producing trans-4-hydroxy-L-proline, which comprises allowing L-proline to coexist with 2-ketoglutaric acid, a divalent iron ion and an enzyme source which catalyzes hydroxylation of L-proline at the 4-position of L-proline in an aqueous medium to convert L-proline into trans-4-hydroxy-L-proline; and recovering the trans-4-hydroxy-L-proline from the aqueous medium.

2. The process according to Claim 1, wherein the enzyme source is derived from a microorganism belonging to the genus Dactylosporangium or Amycolatopsis.

3. The process according to Claim 2, wherein the enzyme source is selected from the group consisting of cells, a culture and processed cells, of the microorganism.

4. The process according to Claim 2, wherein the conversion is carried out during cultivation of the microorganism.

5. The process according to Claim 1, wherein the enzyme source is an L-proline-4-hydroxylase having the following physicochemical properties:
(1) Activity and Substrate Specificity:
The enzyme catalyzes hydroxylation of L-proline at the 4-position of L-proline in the presence of 2-ketoglutaric acid and a divalent iron ion to produce trans-4-hydroxy-L-proline.
(2) Optimum pH Range:
The enzyme has an optimum pH range of 6.0 to 7.0 for its reaction at 30°C for 20 minutes.
(3) Stability pH Range:
The enzyme is stable at pH values of 6.5 to 10.0, when it is allowed to stand at 4°C for 24 hours.
(4) Optimum Temperature Range:
The optimum temperature range is 30 to 40°C when it is allowed to stand at pH 6.5 for 15 minutes.
(5) Stability Temperature Range:
The enzyme is inactivated, when it is allowed to stand at pH 9.0 and at 50°C for 30 minutes.
(6) Inhibitors:
The enzyme is inhibited by Zn⁺⁺ and Cu⁺⁺ and ethylenediaminetetraacetic acid.
(7) Activation:
The enzyme does not need any cofactor for its activity. L-Ascorbic acid accelerates the activity of the enzyme.
(8) Km Value:
Km value is 0.27 mM for L-proline and is 0.55 mM for 2-ketoglutaric acid, when determined in a 80 mM 2-(N-morpholino)ethanesulfonic acid (MES) buffer (pH 6.5) containing 4 mM L-ascorbic acid, 2 mM ferrous sulfate and a pre-determined amount of the enzyme.
(9) Molecular Weight:
The enzyme has a molecular weight of 32,000 ± 5,000 daltons as determined by sodium dodecylsulfate-polyacrylamide gel electrophoresis and of 43,800 ± 5,000 daltons as determined by gel filtration.
(10) N-terminal Amino Acid Sequence:
The enzyme has an N-terminal amino acid sequence illustrated by Sequence Number 1:

6. An L-proline-4-hydroxylase having the following physicochemical properties:
(1) Action and Substrate Specificity:
It catalyzes hydroxylation of L-proline at the 4-position of L-proline in the presence of 2-ketoglutaric acid and a divalent iron ion to produce trans-4-hydroxy-L-proline.
(2) Optimum pH Range:
The enzyme has an optimum pH range of 6.0 to 7.0 for its reaction at 30°C for 20 minutes.
(3) Stable pH Range:
The enzyme is stable at pH values of 6.5 to 10.0, when it is allowed to stand at 4°C for 24 hours.
(4) Optimum Temperature Range:
The optimum temperature range is 30 to 40°C when it is allowed to stand at pH 6.5 for 15 minutes.
(5) Stability Temperature Range:
The enzyme is inactivated, when it is allowed to stand at pH 9.0 and at 50°C for 30 minutes.
(6) Inhibitors:
The enzyme is inhibited by Zn⁺⁺ and Cu⁺⁺ and ethylenediaminetetraacetic acid.
(7) Activation:
The enzyme does not need any cofactor for its activity. L-Ascorbic acid accelerates the reaction.
(8) Km Value:
Km value is 0.27 mM for L-proline and is 0.55 mM for 2-ketoglutaric acid, when determined in a 80 mM 2-(N-morpholino)ethanesulfonic acid (MES) buffer (pH 6.5) containing 4 mM L-ascorbic acid, 2 mM ferrous sulfate and a pre-determined amount of the enzyme.
(9) Molecular Weight:
The enzyme has a molecular weight of 32,000 ± 5,000 daltons as determined by sodium dodecylsulfate-polyacrylamide gel electrophoresis and of 43,800 ± 5,000 daltons as determined by gel filtration.
(10) N-terminal Amino Acid Sequence:
The enzyme has an N-terminal amino acid sequence illustrated by Sequence Number 1

7. A process for producing an L-proline-4-hydroxylase, which comprises cultivating a microorganism belonging to the genus Dactylosporangium or Amycolatopsis and having an ability to produce an L-proline-4-hydroxylase so as to produce and accumulate an L-proline-4-hydroxylase in the culture and recovering the L-proline-4-hydroxylase therefrom.

## Patentansprüche

1. Verfahren zur Herstellung von trans-4-Hydroxy-L-prolin, umfassend das gleichzeitige Vorhandensein von L-Prolin, 2-Ketoglutarsäure, einem divalenten Eisenion und einer Enzymquelle, die eine Hydroxylierung von L-Prolin an der 4-Position von L-Prolin katalysiert, in einem wässrigen Medium, um L-Prolin in trans-4-Hydroxy-L-prolin umzusetzen und Gewinnen von trans-4-Hydroxy-L-prolin aus dem wässrigen Medium.

2. Verfahren gemäß Anspruch 1, wobei die Enzymquelle aus einem Mikroorganismus stammt, der zu der Gattung *Dactylosporangium* oder *Amycolatopsis* gehört.

3. Verfahren gemäß Anspruch 2, wobei die Enzymquelle ausgewählt ist aus der Gruppe bestehend aus Zellen, einer Kultur und behandelten Zellen des Mikroorganismus.

4. Verfahren gemäß Anspruch 2, wobei die Umsetzung während einer Kultivierung des Mikroorganismus erfolgt.

5. Verfahren gemäß Anspruch 1, wobei die Enzymquelle eine L-Prolin-4-hydroxylase mit den nachstehenden physikalisch-chemischen Eigenschaften ist:
(1) Aktivität und Substratspezifität:
Das Enzym katalysiert die Hydroxylierung von L-Prolin an der 4-Position von L-Prolin in Gegenwart von 2-Ketoglutarsäure und einem divalenten Eisenion und bildet trans-4-Hydroxy-L-prolin.
(2) Optimaler pH-Bereich:
Das Enzym hat einen optimalen pH-Bereich von 6,0 bis 7,0 für seine Reaktion bei 30°C und 20 Minuten.
(3) Stabilitäts-pH-Bereich:
Das Enzym ist bei pH-Werten von 6,5 bis 10,0 stabil, falls es 24 Stunden bei 4°C stehengelassen wird.
(4) Optimaler Temperaturbereich:
Der optimale Temperaturbereich ist 30 bis 40°C, falls es 15 Minuten bei pH 6,5 stehengelassen wird.
(5) Stabilitäts-Temperaturbereich:
Das Enzym wird inaktiviert, falls es 30 Minuten bei pH 9,0 und 50°C stehengelassen wird.
(6) Inhibitoren:
Das Enzym wird durch Zn²⁺ und Cu²⁺ und Ethylendiaminotetraessigsäure inhibiert.
(7) Aktivierung:
Das Enzym benötigt für seine Aktivität keinen Co-Faktor. L-Ascorbinsäure erhöht die Aktivität des Enzyms.
(8) Kₘ-Wert:
Der Kₘ-Wert beträgt 0,27 mM für L-Prolin und 0,55 mM für 2-Ketoglutarsäure, falls er in einem 80 mM 2-(N-Morpholino)-ethansulfonsäure (MES)-Puffer (pH 6,5) mit 4 mM L-Ascorbinsäure, 2 mM Eisensulfat und einer vorbestimmten Menge des Enzyms bestimmt wird.
(9) Molekulargewicht:
Das Enzym hat ein Molekulargewicht von 32.000 ± 5000 Dalton, bestimmt durch Natriumdodecylsulfat-Polyacrylamid-Gelelektrophorese und ein Molekulargewicht von 43.800 ± 5000 Dalton, bestimmt durch Gelfiltration.
(10) N-terminale Aminosäuresequenz:
Das Enzym besitzt die in der Sequenz Nr. 1 gezeigte N-terminale Aminosäuresequenz:

6. L-Prolin-4-hydroxylase mit den nachstehenden physikalischen Eigenschaften:
(1) Aktivität und Substratspezifität:
Das Enzym katalysiert die Hydroxylierung von L-Prolin an der 4-Position von L-Prolin in Gegenwart von 2-Ketoglutarsäure und einem divalenten Eisenion und bildet trans-4-Hydroxy-L-prolin.
(2) Optimaler pH-Bereich:
Das Enzym hat einen optimalen pH-Bereich von 6,0 bis 7,0 für seine Reaktion bei 30°C und 20 Minuten.
(3) Stabilitäts-pH-Bereich:
Das Enzym ist bei pH-Werten von 6,5 bis 10,0 stabil, falls es 24 Stunden bei 4°C stehengelassen wird.
(4) Optimaler Temperaturbereich:
Der optimale Temperaturbereich ist 30 bis 40°C, falls es 15 Minuten bei pH 6,5 stehengelassen wird.
(5) Stabilitäts-Temperaturbereich:
Das Enzym wird inaktiviert, falls es 30 Minuten bei pH 9,0 und 50°C stehengelassen wird.
(6) Inhibitoren:
Das Enzym wird durch Zn⁺⁺ und Cu⁺⁺ und Ethylendiaminotetraessigsäure inhibiert.
(7) Aktivierung:
Das Enzym benötigt für seine Aktivität keinen Co-Faktor. L-Ascorbinsäure beschleunigt die Reaktion.
(8) Kₘ-Wert:
Der Kₘ-Wert beträgt 0,27 mM für L-Prolin und 0,55 mM für 2-Ketoglutarsäure, falls er in einem 80 mM 2-(N-Morpholino)-ethansulfonsäure (MES)-Puffer (pH 6,5) mit 4 mM L-Ascorbinsäure, 2 mM Eisensulfat und einer vorbestimmten Menge des Enzyms bestimmt wird.
(9) Molekulargewicht:
Das Enzym hat ein Molekulargewicht von 32.000 ± 5000 Dalton, bestimmt durch Natriumdodecylsulfat-Polyacrylamid-Gelelektrophorese und ein Molekulargewicht von 43.800 ± 5000 Dalton, bestimmt durch Gelfiltration.
(10) N-terminale Aminosäuresequenz:
Das Enzym besitzt die in der Sequenz Nr. 1 gezeigte N-terminale Aminosäuresequenz:

7. Verfahren zur Herstellung einer L-Prolin-4-hydroxylase, umfassend die Kultivierung eines Mikroorganismus, der zur Gattung *Dactylosporangium* oder *Amycolatopsis* gehört und zur Herstellung einer L-Prolin-4-hydroxylase fähig ist, so dass L-Prolin-4-hydroxylase in der Kultur produziert und akkumuliert wird, und Gewinnen der L-Prolin-4-hydroxylase aus der Kultur.

## Revendications

1. Procédé de production de trans-4-hydroxy-L-proline, qui comporte le fait de mettre de la L-proline en présence d'acide 2-cétoglutarique, d'ions de fer divalent et d'une source d'une enzyme qui catalyse l'hydroxylation de la L-proline en position 4, dans un milieu aqueux, afin de convertir la L-proline en trans-4-hydroxy-L-proline, et le fait de récupérer la trans-4-hydroxy-L-proline formée dans le milieu aqueux.

2. Procédé conforme à la revendication 1, dans lequel la source d'enzyme dérive d'un microorganisme appartenant au genre Dactylosporangium ou Amycolatopsis.

3. Procédé conforme à la revendication 2, dans lequel la source d'enzyme est choisie dans l'ensemble constitué par des cellules, une culture et des cellules traitées du microorganisme.

4. Procédé conforme à la revendication 2, dans lequel la conversion est effectuée au cours d'une opération de culture du microorganisme.

5. Procédé conforme à la revendication 1, dans lequel la source d'enzyme est une L-proline 4-hydroxylase qui possède les propriétés physicochimiques suivantes:
a) Activité et substrat spécifique :
Cette enzyme catalyse l'hydroxylation de la L-proline au niveau de sa position 4, en présence d'acide 2-cétoglutarique et d'ions de fer divalent, réaction qui donne de la trans-4-hydroxy-L-proline ;
b) Intervalle de pH optimal :
L'intervalle de pH optimal pour faire réagir cette enzyme pendant 20 minutes à 30 °C va de 6,0 à 7,0 ;
c) Intervalle de pH de stabilité :
Cette enzyme est stable à un pH de 6,5 à 10,0, quand on la laisse au repos à 4 °C pendant 24 heures ;
d) Intervalle de température optimal :
L'intervalle de température optimal va de 30 à 40 °C, quand on laisse cette enzyme au repos pendant 15 minutes à pH 6,5 ;
e) Intervalle de température de stabilité :
Cette enzyme est désactivée quand on la laisse au repos à pH 9,0 et à 50 °C pendant 30 minutes ;
f) Inhibiteurs :
Cette enzyme est inhibée par les ions Zn²⁺ et Cu²⁺, ainsi que par l'acide éthylènediamine-tétracétique ;
g) Activation :
Pour exercer son activité, cette enzyme n'a besoin d'aucun cofacteur, mais l'acide L-ascorbique augmente son activité ;
h) Valeurs de Kₘ :
Kₘ vaut 0,27 mM pour la L-proline et 0,55 mM pour l'acide 2-cétoglutarique, valeurs déterminées dans une solution tampon à 80 mM de MES [acide 2-(N-morpholino)éthanesulfonique] (pH 6,5) et contenant 4 mM d'acide L-ascorbique, 2 mM de sulfate ferreux et une quantité connue de cette enzyme ;
i) Masse moléculaire :
La masse moléculaire de cette enzyme, mesurée par électrophorèse sur gel de polyacrylamide en présence de dodécylsulfate de sodium, vaut 32 000 ± 5 000 daltons, mais déterminée par filtration sur gel, elle vaut 43 800 ± 5 000 daltons ;
j) Séquence N-terminale d'acides aminés
La séquence N-terminale d'acides aminés de cette enzyme est présentée ci-dessous dans la Séquence n° 1 :

6. L-proline 4-hydroxylase possédant les propriétés physico-chimiques suivantes :
a) Activité et substrat spécifique :
Cette enzyme catalyse l'hydroxylation de la L-proline au niveau de sa position 4, en présence d'acide 2-cétoglutarique et d'ions de fer divalent, réaction qui donne de la trans-4-hydroxy-L-proline ;
b) Intervalle de pH optimal :
L'intervalle de pH optimal pour faire réagir cette enzyme pendant 20 minutes à 30 °C va de 6,0 à 7,0 ;
c) Intervalle de pH de stabilité :
Cette enzyme est stable à un pH de 6,5 à 10,0, quand on la laisse au repos à 4 °C pendant 24 heures ;
d) Intervalle de température optimal :
L'intervalle de température optimal va de 30 à 40 °C, quand on laisse cette enzyme au repos pendant 15 minutes à pH 6,5 ;
e) Intervalle de température de stabilité :
Cette enzyme est désactivée quand on la laisse au repos à pH 9,0 et à 50 °C pendant 30 minutes ;
f) Inhibiteurs :
Cette enzyme est inhibée par les ions Zn²⁺ et Cu²⁺, ainsi que par l'acide éthylènediamine-tétracétique ;
g) Activation :
Pour exercer son activité, cette enzyme n'a besoin d'aucun cofacteur, mais l'acide L-ascorbique en accélère la réaction ;
h) Valeurs de Kₘ :
Kₘ vaut 0,27 mM pour la L-proline et 0,55 mM pour l'acide 2-cétoglutarique, valeurs déterminées dans une solution tampon à 80 mM de MES [acide 2-(N-morpholino)éthanesulfonique] (pH 6,5) et contenant 4 mM d'acide L-ascorbique, 2 mM de sulfate ferreux et une quantité connue de cette enzyme ;
i) Masse moléculaire :
La masse moléculaire de cette enzyme, mesurée par électrophorèse sur gel de polyacrylamide en présence de dodécylsulfate de sodium, vaut 32 000 ± 5 000 daltons, mais déterminée par filtration sur gel, elle vaut 43 800 ± 5 000 daltons ;
j) Séquence N-terminale d'acides aminés :
La séquence N-terminale d'acides aminés de cette enzyme est présentée ci-dessous dans la Séquence n° 1 :

7. Procédé de production d'une L-proline 4-hydroxylase, qui comporte le fait de cultiver un microorganisme appartenant au genre Dactylosporangium ou Amycolatopsis et capable de produire une L-proline 4-hydroxylase, de façon qu'une L-proline 4-hydroxylase soit produite et s'accumule dans la culture, et le fait de récupérer là-dedans cette L-proline 4-hydroxylase.
